(19) Europäisches Patentamt
European Patent Office
Office européen des brevets

(11) Veröffentlichungsnummer : **0 050 325**
**B1**

(12) # EUROPÄISCHE PATENTSCHRIFT

(45) Veröffentlichungstag der Patentschrift : **17.07.85**

(51) Int. Cl.⁴ : **C 07 J 9/00**, C 07 J 13/00, C 07 J 53/00, C 07 J 31/00

(21) Anmeldenummer : **81108386.4**

(22) Anmeldetag : **15.10.81**

(54) **Verfahren zur Herstellung von Zwischenprodukten in der Synthese von Vitamin D3-Metaboliten und neue Zwischenprodukte.**

(30) Priorität : **22.10.80 US 199167**

(43) Veröffentlichungstag der Anmeldung : **28.04.82 Patentblatt 82/17**

(45) Bekanntmachung des Hinweises auf die Patenterteilung : **17.07.85 Patentblatt 85/29**

(84) Benannte Vertragsstaaten : **AT BE CH DE FR GB IT LI NL**

(56) Entgegenhaltungen :
**DE-A- 2 746 107**
**GB-A- 666 106**
**US-A- 3 946 052**
**CHEMISCHE BERICHTE, Band 98, Heft 2, 1965, Seiten 604-612 WEINHEIM (DE) G. DREFAHL et al.: "Synthesen von Alkyliden-Steroiden durch Wittig-Reaktion".**
**JOURNAL OF ORGANIC CHEMISTRY, Band 25, Nr. 1, 29. Februar 1960, Seiten 79-83 WASHINGTON D.C. (US) J.P. DUSZA et al.: "20-Methyl-pregnane and derivatives"**
**Die Pharmazie - Vol. 30(1975), S. 30-31**
**J. of Organic Chemistry, Vol. 36, Nr. 17( 1971), S. 2403-2406**
**J.A.C.S. 1951, S. 5073-5076**

(73) Patentinhaber : **F. HOFFMANN-LA ROCHE & CO. Aktiengesellschaft**
**CH-4002 Basel (CH)**

(72) Erfinder : **Batcho, Andrew D.**
**19 White Oak Drive**
**North Caldwell, N.J. 07006 (US)**
Erfinder : **Berger, Donald E. Jr.**
**181 Del Medio Avenue**
**Mountain View, Calif. 94040 (US)**
Erfinder : **Uskokovic, Milan R.**
**253 Highland Avenue**
**Upper Montclair, N.J. 07043 (US)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

**EP 0 050 325 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von Zwischenprodukten in der Synthese von Vitamin $D_3$-Metaboliten, wie 1,25-Dihydroxy-, 25-Hydroxy- und 24R,25-Dihydroxycholecalciferol, ausgehend von 17-Ketosteroiden, über Zwischenprodukte mit natürlicher α-Orientierung am Kohlenstoffatom in 20-Stellung des Steroidgerüsts.

Die Erfindung betrifft insbesondere ein Verfahren zur Herstellung einer Verbindung der Formel

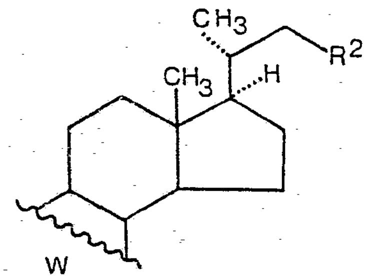

(I)

worin W eine Gruppierung der Formel

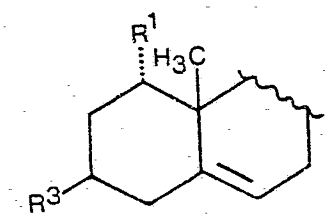

W¹

oder

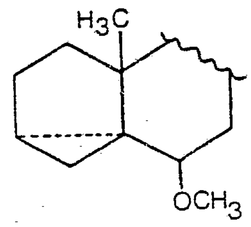

W²

$R^1$ Wasserstoff, Hydroxy oder Acyloxy, $R^2$ und $R^3$ Hydroxy oder Acyloxy, wobei wenn $R^2$ Acyloxy ist, $R^1$ und $R^3$ von Hydroxy verschieden sind, welches dadurch gekennzeichnet ist, dass man

A) eine Verbindung der Formel

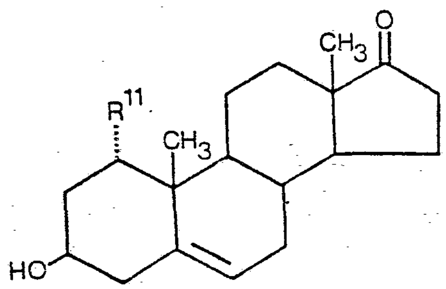

(II)

worin $R^{11}$ Wasserstoff oder Hydroxy ist, in einer Wittig-Reaktion mit einem Aethyltriphenylphosphoniumhalogenid zu einer Verbindung der Formel

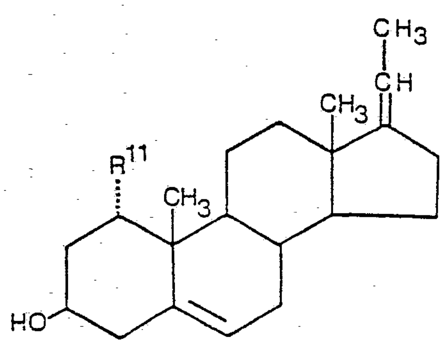

(IIIa)

worin $R^{11}$ die obige Bedeutung hat, umsetzt,

B) a) gewünschtenfalls die in einem Steroid der Formel IIIa enthaltenen 1α- und/oder 3β-Hydroxygruppen acyliert und gewünschtenfalls die Acylgruppe in 3-Stellung in einem erhaltenen diacylierten Steroid der Formel IIIa selektiv hydrolysiert, b) gewünschtenfalls die Hydroxygruppe in einer Verbindung der Formel IIIa, worin $R^{11}$ Wasserstoff ist, zu einer reaktionsfähigeren Abgangsgruppe umwandelt,

C) das Produkt von Stufe A) oder B) der Formel

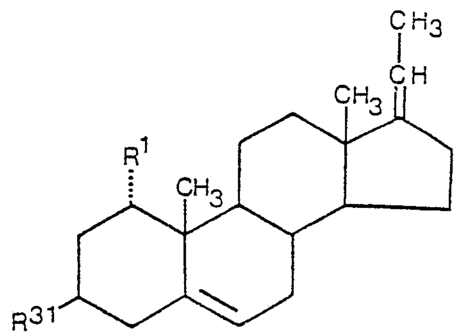

(IIIb)

worin $R^1$ die obige Bedeutung hat und $R^{31}$ Hydroxy, Acyloxy oder, falls $R^1$ Wasserstoff ist, auch eine Abgangsgruppe darstellt, mit Formaldehyd, gewünschtenfalls in Gegenwart eines Acylierungsmittels, zu einer Verbindung der Formel

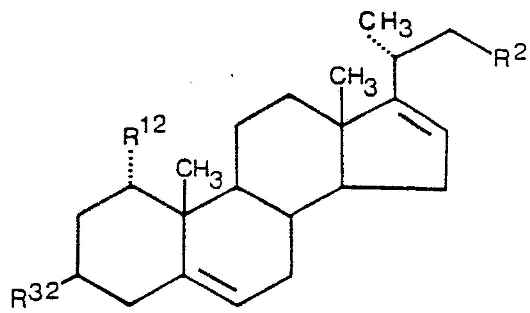

(IVa)

worin entweder $R^2$ Hydroxy ist und $R^{12}$ und $R^{32}$ die Bedeutungen von $R^1$ bzw. $R^{31}$ haben oder $R^2$ Acyloxy, $R^{12}$ Wasserstoff oder Acyloxy und $R^{32}$ Acyloxy oder eine Abgangsgruppe darstellen, umsetzt,

D) gewünschtenfalls eine Verbindung der Formel

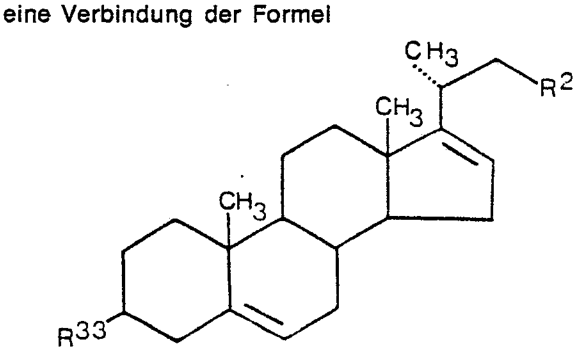

(IVb)

worin $R^2$ die obige Bedeutung hat und $R^{33}$ eine Abgangsgruppe ist, mit einer schwachen Base und Methanol zu einer Verbindung der Formel

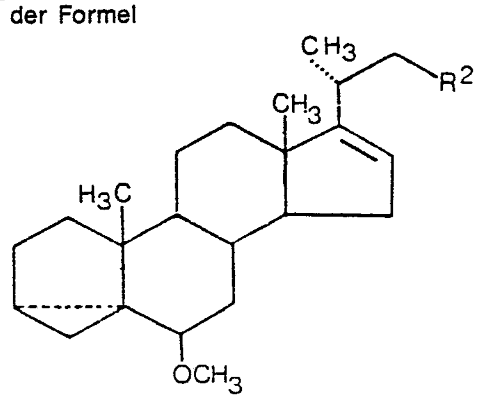

(IVc)

worin $R^2$ die obige Bedeutung hat, umsetzt und

E) das Produkt von Stufe C) oder D) der Formel

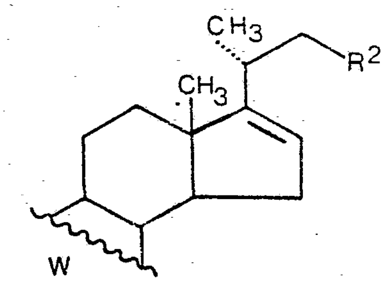

(IV)

worin W und $R^2$ die obige Bedeutung haben, katalytisch hydriert.

Der Ausdruck Niederalkyl bezieht sich auf geradkettige oder verzweigte gesättigte aliphatische Kohlenwasserstoffgruppen, vorzugsweise mit 1-7 Kohlenstoffatomen, wie Methyl, Aethyl, Isopropyl, Butyl, Pentyl, Hexyl, Heptyl und Octyl. Der Ausdruck Niederalkoxy bezeichnet die oben definierte Alkylgruppen die über ein Sauerstoffatom an den Rest des Moleküls verbunden sind. Der Ausdruck Aryl bezeichnet eine aromatische Gruppe wie Phenyl, die durch eine oder mehrere Alkylgruppen substituiert sein kann, wie Tolyl. Der Ausdruck Acyloxy bezeichnet den durch Abspaltung des Wasserstoffatoms aus der Carboxygruppe einer Carbonsäure verbleibenden Rest. Beispiele von solchen Carbonsäuren sind Ameisensäure, Essigsäure, Pivalinsäure, Propionsäure, Buttersäure, Capronsäure, Oenanthsäure, Undecylensäure und Oelsäure, sowie aromatische Carbonsäuren mit 7-15 Kohlenstoffatomen, wie Benzoesäure oder Phenylessigsäure. Bevorzugte Acyloxygruppen sind $C_{1-7}$-Alkanoyloxygruppen, insbesondere Acetoxy, die mit einem oder mehreren Halogenatomen substituiert sein können. Unter den bevorzugten halosubstituierten Niederalkanoyloxygruppen sind die Trihaloacetoxygruppen, insbesondere Trifluoracetoxy, zu nennen.

Die Stufe A) des Verfahrens kann unter den für Wittig-Reaktionen üblichen Bedingungen durchgeführt werden, zweckmässig unter einer inerten Atmosphäre bei Temperaturen zwischen etwa 0 und 150 °C, vorzugsweise bei Raumtemperatur, in einem inerten organischen Lösungsmittel, wie einem inerten aprotischen organischen Lösungsmittel, z. B. Tetrahydrofuran, Dimethylsulfoxid, Dimethylformamid, Benzol, Toluol oder Hexan, in Gegenwart einer starken Base, wie einem Niederalkylalkalimetallsalz, z. B. Butyllithium, einem Alkoholsatz, wie Kalium-tert.-Butylat oder Kaliumamylat.

Die Acylierung in 3-Stellung einer Verbindung der Formel IIIa kann selektiv durchgeführt werden, indem man 1 Mol einer Verbindung der Formel IIIa mit 1 Mol Acylierungsmittel umsetzt. Die Acylierung in den Stellungen 1 und 3 einer Verbindung der Formel IIIa, worin $R^{11}$ Hydroxy ist, kann dadurch bewerkstelligt werden, dass man 1 Mol einer solchen Verbindung mit 2 Mol des Acylierungsmittels umsetzt. Beispiele von Acylierungsmitteln sind Niederalkancarbonsäureanhydride, wie Essigsäure oder Trifluoressigsäureanhydrid, aromatische Anhydride, wie Benzoesäure, Nitrobenzoesäure oder Toluylsäureanhydrid. Die Acylierung wird zweckmässig in Gegenwart einer schwachen Base, wie Pyridin, Dimethylanilin, Triäthylamin oder Natriumacetat, und gewünschtenfalls in Gegenwart von Dimethylaminopyridin, welches die Reaktion katalysiert, durchgeführt.

Zur Herstellung eines Steroids der Formel IIIa, worin nur die Hydroxygruppe in 1-Stellung acyliert ist, kann man ein Steroid der Formel IIIa, worin $R^{11}$ Hydroxy ist, wie oben beschrieben in den Stellungen 1 und 3 acylieren und 1 Mol des so erhaltenen Produktes bei Rückflusstemperatur mit 1 Mol einer Base umsetzen, wobei die Acylgruppe in Stellung 3 selektiv hydrolysiert wird.

In einer Variante b) der Stufe B) der Verfahrens kann die Hydroxygruppe in 3-Stellung einer Verbindung der Formel IIIa, worin $R^{11}$ Wasserstoff ist, in herkömmlicher Weise in eine reaktionsfähigere Abgangsgruppe umgewandelt werden. Beispiele von solchen Abgangsgruppen sind Halogenide, Niederalkyl- oder Arylsulfonyloxy, vorzugsweise Arylsulfonyloxy, wobei p-Toluolsulfonyloxy besonders bevorzugt ist. Die Reaktion kann unter einer inerten Atmosphäre in Gegenwart einer schwachen Base, wie Pyridin, bei Raumtemperatur durchgeführt werden.

In der Schlüsselstufe C) des Verfahrens wird eine Verbindung der Formel IIIb mit Formaldehyd umgesetzt. Die Reaktion kann in einem herkömmlichen inerten Lösungsmittel, wie Methylenchlorid, Tetrachlorkohlenstoff oder Chloroform, einen aromatischen Kohlenwasserstoff, wie Benzol oder Toluol oder einem niederen aliphatischen Kohlenwasserstoff, wie Hexan oder Octan, oder auch in wässrigem Medium mittels protischen Säuren durchgeführt werden. Die Reaktionstemperatur ist nicht kritisch. Zweckmässig wird bei einer Temperatur zwischen etwa – 20 und 45 °C gearbeitet. Die Reaktion kann durch Lewis oder protische Säuren, wie Bortrifluoridätherat, Aluminiumchlorid, Methansulfonsäure und Trifluoressigsäure, vorzugsweise Bortrifluoridätherat, katalisiert werden. Anstelle von Formaldehyd kann man eine Verbindung verwenden aus der Formaldehyd in situ gebildet wird, z. B. Paraformaldehyd.

Falls man die Stufe C) in Gegenwart eines Acylierungsmittels durchführt, wird eine Verbindung der Formel IVa worin $R^2$ Acyloxy ist, erhalten.

Die Stufe D) wird zweckmässig bei Rückflusstemperatur des Reaktionsgemisches durchgeführt, wobei wasserfreies Pyridin die bevorzugte schwache Base ist.

Die selektive Hydrierung von Stufe E) wird in einer Wasserstoffatmosphäre in Gegenwart eines Hydrierungskatalysators, vorzugsweise Platin auf Aktivkohle, z. B. 5 % Platin auf Aktivkohle, Platinoxid und Raney-Nickel durchgeführt. Die Reaktion wird zweckmässig in einem inerten Lösungsmittel unter einer Wasserstoffatmosphäre bei einer Temperatur zwischen etwa 0 und 40 °C durchgeführt.

Die Verfarensprodukte der Formel I können zur Herstellung von Verbindungen, wie 1,25-Dihydroxy-, 25-Hydroxy- und 24R,25-Dihydroxycholesterin verwendet werden. Diese Verbindungen können zur Herstellung der oben erwähnten Vitamin $D_3$-Metaboliten verwendet werden.

Zur Beispiel kann 1α,25-Dihydroxycholesterol dadurch hergestellt werden, dass man eine Verbindung der Formel I, worin W die Bedeutung von $W^1$ hat, $R^1$ und $R^3$ Acyloxy und $R^2$ Hydroxy bedeuten, z. B. (20S)-20-Methylpregn-5-en-1α,- 3β,21-triol-1,3-diacetat mit Paratoluolsulfonylchlorid in Gegenwart einer schwachen Base wie Pyridin bei Temperaturen zwischen etwa − 10 und 40 °C zur entsprechenden Verbindung der Formel I, worin $R^2$ p-Toluolsulfonyloxy, wie (20S)-1α,- 3β-Diacetoxy-20-methyl-21-p-toluolsulfonyloxypregn-5-en umsetzt.

Diese Verbindung wird mit Lithiumaluminiumhydrid in Gegenwart eines inerten aprotischen Lösungsmittels, wie Tetrahydrofuran, bei einer Temperatur zwischen etwa − 20 und 0 °C zu (20S)-1α,3β-Dihydroxy-20-methyl-21-p-toluolsulfonyloxypregn-5-en umgesetzt. Diese Verbindung wird mit einer herkömmlichen eine Hydroxyschutzgruppe abgebenden Verbindung, wie 3,4-Dihydro-2H-pyran und wasserfreier p-Toluolsulfonsäure gefolgt von Natriumbicarbonat, zu (20S)-1α,3β-bis [(Tetrahydro-2H-pyran-2-yl)-oxy]-20-methyl-21-p-toluolsulfonyloxypregn-5-en umgesetzt. Letzeres kann wie in der DOS 3 104 948 beschrieben in 1α,25-Dihydroxycholesterin übergeführt werden.

In einer Variante der Herstellung des 1α,25-Dihydroxycholesterins wird eine triacylierte Verbindung der Formel I, wie (20S)-20-Methyl-1α,3β,21-triacetoxypregn-5-en, mit einer starken Base, wie Natriumhydroxid, unter Rückfluss zu (20S)-20-Methylpregn-5-en-1α,3β,21-triol umgesetzt. Dieses wird dann selektiv in 21-Stellung acyliert, z. B. durch Umsetzung mit Essigsäureanhydrid in Gegenwart von Bleidiacetat-trihydrat in einer schwachen Base, wie Dimethylformamid, bei Raumtemperatur. Die erhaltene Verbindung, z. B. (20S)-21-Acetoxy-20-methylpregn-5-en-1α,3β-diol, wird mit einem herkömmlichen eine Hydroxyschutzgruppe abgebenden Reagenz zu einer Verbindung, wie (20S)-21-Acetoxy-1α,3β-bis-[(tetrahydro-2H-pyran-2-yl)-oxy]-20-methyl-pregn-en, umgesetzt. Zur Hydrolyse der Acyloxygruppe in einer solchen Verbindung wird letztere mit einer Base, wie einer methanolischen Kaliumhydroxidlösung, bei einer Temperatur zwischen etwa − 20 und 100 °C umgesetzt und man erhält eine Verbindung, wie (20S)-1α,3β-bis[(tetrahydro-2H-pyran-2-yl)oxy]-20-methylpregn-5-en-21-ol, welches dann durch Reaktion mit Paratoluolsulfonylchlorid in Gegenwart einer schwachen Base, wie Pyridin, bei einer Temperatur zwischen etwa − 10 und 40 °C zu einer Verbindung, wie das oben erwähnte (20S)-1α,3β-bis[(Tetrahydro-2H-pyran-2-yl)-oxy]-20-methyl-21-p-toluolsulfonyloxypregn-5-en übergeführt wird.

21-Hydroxycholesterin und 24R,25-Dihydroxycholesterin können ausgehend von einer Verbindung der Formel I, worin $R^1$ Wasserstoff und $R^3$ Acyloxy sind, z. B. wie beschrieben in Y. Fujimoto et al., J. Chem. Soc., Perkin I, 1975, 2302 und R. R. Muccino et al., Steroids, 36, 645 (1978), sowie in den US Patenten 3 822 254 und 4 038 272, hergestellt werden.

Die Zwischenprodukte der Formel

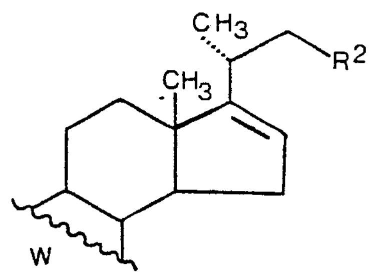

(V)

worin W und $R^2$ die obige Bedeutung haben, sowie diejenigen der Formel

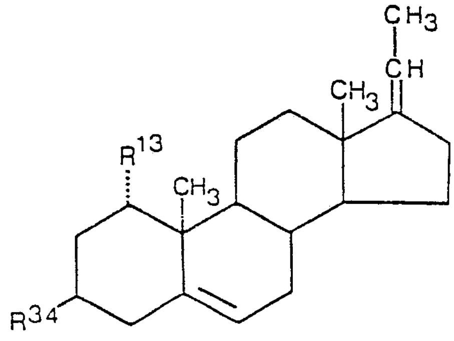

(IIIc)

worin $R^{13}$ und $R^{34}$ Hydroxy oder Trifluoracetoxy oder $R^{13}$ Wasserstoff und $R^{34}$ Halogen, Niederalkylsulfonyloxy oder Arylsulfonyloxy sind, sowie die Verbindungen (20S)-1α,3β-Bistrifluoracetoxy-20-methylpregn-5-en-21-ol und (20S)-20-Methyl-1α,3β-bistrifluoracetoxypregn-5-en-21-ol-p-toluolsulfonat, die ausgehend von den oben erwähnten Zwischenprodukten hergestellt werden können, sind neu als solche Gegenstand der vorliegenden Erfindung.

In Chem. Ber. *98*, 1965, 604 wird die Ueberführung des 3β-Hydroxy-$\Delta^5$-androsten-17-ons in einer Wittig-Reaktion in das 17-Aethyliden-$\Delta^5$-androsten-3β-ol nicht aber die erfindungsgemässe Ueberführung der 3β-Hydroxy-$\Delta^5$-androsten-17-one der Formel II in die 21-substituierten Pregnane der Formel I offenbart.

Einige dieser Pregnanen der Formel I fallen unter die Formel I in der DE-A-2,746,107. Ferner fällt die Verbindung IIa auf Seite 81 in J. Org. Chem. *25*, 1960 unter die vorliegende Formel I. Jedoch ist das erfindungsgemässe Verfahren zur Herstellung der Verbindungen der Formel I völlig verschieden von den in der besagten DE-A und dem Artikel beschriebenen Verfahren.

In GB-A-666,106, Pharmazie *30*, (1975), 30, J.A.C.S. (1951), 5073, J. Org. Chem. 36 (1971), 2403 und Chem. Ber. *98*, (1965), 604 sind das 3β-Hydroxy- und das 3β-Acetoxy-pregna-5,17(20)-dien beschrieben, nicht aber die erfindungsgemässen Verbindungen der Formel IIIc.

## Beispiel 1

Einer Suspension von 9,12 g (30 mMol) 1α-Hydroxydehydroepiandrosteron und 25,1 g (60 mMol) Aethyltriphenylphosphoniumjodid in 250 ml Tetrahydrofuran werden unter Rühren unter einer Stickstoffatmosphäre 6,7 g (60 mMol) Kalium tert.-Butoxyd zugefügt. Das Gemisch wird bei Raumtemperatur gerührt und dann unter Rückfluss erhitzt. Man erhält (Z)-1α,3β-Pregn-5,17(20)-dien-1,3-diol.

Das Gemisch wird auf 50 °C abgekühlt und mit 60 ml Pyridin und 30 ml Essigsäureanhydrid versetzt. Die Lösung wird gerührt und dann unter Rückfluss erhitzt. Da die Acetylierung unvollständig ist, werden 100 mg 4-Dimethylaminopyridin zugesetzt und das Erhitzen auf Rückflusstemperatur fortgeführt. Die Lösung wird mit wässrigem Methanol verdünnt und nach Abkühlen auf Raumtemperatur mit Hexan extrahiert. Die organischen Phasen werden mit wässrigem Methanol gewaschen und zu 12 g Rohprodukt eingedampft. Nach Chromatographie auf Silicagel und Elution mit Hexan-Aethylacetat erhält man 6,8 g unreines Produkt und 4,2 g reines Produkt. Das unreine Produkt gibt nach Chromatographie 6,2 g reines Produkt. Man erhält eine Gesamtmenge von 10,4 g (87 %) reines 1α,3β-Diacetoxypregna-5,17(20)-dien in Form eines amorphen Feststoffes und als 95 : 5-Gemisches der Z- und E-Isomeren. Nach Umkristallisation aus Methanol schmilzt (Z)-1α,3β-Diacetoxypregna-5,17(20)-dien bei 90-91 °C.

## Beispiel 2

Einer Suspension von 4,0 g (10,0 mMol) (Z)-Pregna-5,17(20)-dien-1α,3β-dioldiacetat und 0,90 g (30 mMol Monomer) Paraformaldehyd in 50 ml Methylenchlorid werden unter Rühren unter einer Stickstoffatmosphäre 10 ml einer Lösung von Bortrifluoridätherat in Methylenchlorid (1 : 90, v/v) zugefügt. Dem Gemisch werden dann 100 ml einer gesättigten wässrigen Natriumbicarbonatlösung zugesetzt und das aus zwei Phasen bestehende Gemisch wird filtriert. Das Filtrat wird mit Methylenchlorid extrahiert. Die organischen Phasen werden mit gesättigter wässriger Natriumbicarbonatlösung gewaschen, getrocknet und zu 4,3 g eines festen Schaums eingedampft. Nach Chromatographie auf Silicagel und Elution mit Hexan-Aethylacetat erhält man 3,00 g reines Produkt. Durch Chromatographie der unreinen Fraktionen werden zusätzlich 0,46 g Produkt erhalten. Insgesamt erhält man 3,46 g (81 %) (20S)-20-Methylpregna-5,16-dien-1α,3β,21-triol-1,3-diacetat in Form eines amorphen Feststoffes. Nach Kristallisation aus Hexan-Aethylacetat und Umkristallisation aus Methanol-Wasser schmilzt das Produkt bei 121-122 °C.

## Beispiel 3

Einer Lösung von 860 mg (2,0 mMol) (20S)-20-Methylpregna-5,16-dien-1α,3β,21-triol-1,3-diacetat in 50 ml absolutem Aethanol werden 89 mg 5 % Platin auf Aktivkohle zugesetzt. Die Suspension wird unter einer Wasserstoffatmosphäre bei 23 °C bis die Wasserstoffaufnahme von 48 ml (Theorie 48,8 ml) erreicht, gerührt. Der Katalysator wird abfiltriert und mit Methylenchlorid gewaschen. Das Filtrat wird eingedampft und der Rückstand, 835 mg (87 %), wird aus 20 ml Hexan kristallisiert. Man erhält 746 mg (86 %) (20S)-20-Methylpregn-5-en-1α,3β,21-triol-1,3-diacetat in Form von weissen Kristallen, Smp. 133,5-134,5 °C.

## Beispiel 4

Einer Lösung von 2.2 g (5,5 mMol) (Z)-Pregna-5,17(20)-dien-1α,3β-dioldiacetat, 0,75 g (2,5 mMol Monomer) Paraformaldehyd und 5 ml (50 mMol) Essigsäureanhydrid in 5 ml Methylenchlorid werden unter einer Argonatmosphäre unter Rühren 5 ml einer Lösung von Bortrifluorid in Methylenchlorid (1 : 90, v/v) zugesetzt. Die Lösung wird auf 500 ml 5 % Natriumbicarbonatlösung geschüttet und gerührt

und danach mit Aethylacetat extrahiert. Die organischen Phasen werden mit gesättigter Natriumbicarbonatlösung und Kochsalzlösung gewaschen, dann getrocknet, filtriert und zu 3,0 g Produkt eingedampft. Nach Chromatographie auf Silicagel und Elution mit Hexan-Aethylacetat erhält man 1,275 g und dann 0,874 g Produkt. Letztere Fraktion wurde in einem Hochdruckflüssigchromatographen mit Hexan-Aethylacetat behandelt, wobei man zusätzlich 0,580 g Produkt erhält. Insgesamt erhält man 1,855 g (71 %) (20S)-20-Methylpregna-5,16-dien-1α,3β,21-trioltriacetat, Smp. 116-118 °C. Nach Umkristallisation aus Methanol und dann aus Acetonitril schmilzt das Produkt bei 119,5-120,5°C.

Beispiel 5

Einer Lösung von 1,370 g (4,0 mMol) (Z)-3β-Acetoxypregna-5,17(20)-dien, 1,20 g (40 mMol) Paraformaldehyd und 8 ml (85 mMol) Essigsäureanhydrid in 16 ml Methylenchlorid werden unter Rühren unter einer Stickstoffatmosphäre 4 ml einer Lösung von Bortrifluoridätherat in Methylenchlorid (1 : 90, v/v) zugesetzt. Der grösste Teil des Methylenchlorids wird eingedampft und die zurückbleibende Lösung in 120 ml gesättigter wässriger Natriumbicarbonatlösung geschüttet und gerührt und dann mit Methylenchlorid extrahiert. Die organische Phase wird filtriert, getrocknet und eingedampft und man erhält 1,76 g Feststoff. Nach Umkristallisation aus Acetonitril erhält man 0,98 g (59 %) (20S)-20-Methylpregna-5,16-dien-3β,21-dioldiacetat, Smp. 116-117°C.

Beispiel 6

50 ml einer alkoholischen Suspension von 414 mg (1 mMol) (20S)-20-Methylpregna-5,16-dien-3β,21-diolacetat und 50 mg 5 % Platin auf Aktivkohle werden unter einer Wasserstoffatmosphäre bis zur Aufnahme von 22,3 ml (Theorie 24 ml) gerührt. Der Katalysator wird abfiltriert und gewaschen. Nach Eindampfen des Filtrats erhält man 413 mg weissen Feststoff, Smp. 121-125 °C. Nach Umkristallisation aus Acetonitril erhält man 298 mg (71 %) (20S)-20-Methylpregn-5-en-3β,21-diolacetat in Form von weissen Kristallen, Smp. 125-127 °C.

Beispiel 7

a) Einer Lösung von 34,2 g (0,10 mMol) (Z)-Pregna-5,-17(20)-dien-3β-olacetat und 9,0 g (0,30 mMol Monomer) Paraformaldehyd in 500 ml Methylenchlorid werden unter Rühren unter einer Stickstoffatmosphäre 91 ml einer Lösung von Bortrifluoridätherat in Methylenchlorid (1 : 90, v/v) zugesetzt. Nach Zusatz von 100 ml gesättigter Natriumbicarbonatlösung wird die Suspension filtriert. Die Methylenchloridphase wird getrocknet und eingedampft. Man erhält 27,1 g Produkt. Nach Chromatographie auf Silicagel und Elution mit Hexan-Aethylacetat (4 : 1) erhält man 31,2 g (84 %) (20S)-20-Methylpregna-5,16-dien-3β,21-diol-3-acetat, Smp. 170-171 °C. Nach Kristallisation aus Aethylacetat schmilzt das Produkt bei 168-170 °C.

b) In zum Verfahren a) analoger Weise werden folgende Katalysatoren verwendet :
Bortrifluoridhydrat, Bortrifluorid-Methanol-Komplex, Bortrifluorid-Aethylamin-Komplex, Aluminiumchlorid, Aluminiumchlorid (und Benzol als Lösungsmittel) Zinkchlorid, Zinnetetachlorid, Bortrichlorid, Titantetrachlorid, Methansulfonsäure, p-Toluolsulfonsäuremonohydrat, Trifluoressigsäure oder Oxalsäuremonohydrat.

c) In zum Verfahren a) analoger Weise werden folgende Lösungsmittel verwendet :
Chloroform, Tetrachlorkohlenstoff, Toluol, Tetrahydrofuran, Diäthyläther oder Essigsäure.

d) In zum Verfahren a) analoger Weise werden Methansulfonsäure, Toluolsulfonsäuremonohydrat oder Schwefelsäure als Katalysatoren und an Stelle des Paraformaldehyds Formalin (wässriges Formaldehyd) verwendet.

Beispiel 8

Einer Suspension von 601 mg (2 mMol) (Z)-Pregna-5,17(20)-dien-3β-ol, 180 mg (3 mMol Monomer) Paraformaldehyd und 10 ml Methylenchlorid werden unter Rühren unter einer Stickstoffatmosphäre 2 ml Bortrifluorodätherat in Methylenchlorid (1 : 90, v/v) zugesetzt. Dann wird gesättigtes Natriumbicarbonat zugesetzt. Die Suspension wird mit Methylenchlorid extrahiert, mit Kochsalzlösung gewaschen, getrocknet und eingedampft. Der Rückstand wird nochmals wie weiter oben beschrieben behandelt. Man erhält 694 mg Feststoff. Nach Chromatographie auf Silicagel und Elution mit Methylenchlorid-Aethylacetat (5 : 1) erhält man 406 mg (61 %) (20S)-20-Methylpregna-5,16-dien-3β,21-diol, Smp. 190-192,5 °C.

Beispiel 9

Eine Lösung von 1,2 g (2,9 mMol) (20S)-20-Methylpregna-5,16-dien-3β,21-dioldiacetat und 0,16 g

Natriumhydroxid in 25 ml Methanol wird bei Rückflusstemperatur erhitzt und dann in 250 ml Wasser gegossen. Die Suspension wird mit Aethylacetat extrahiert, mit Kochsalzlösung gewaschen, getrocknet und eingedampft. Man erhält 0,95 g Feststoff. Nach Umkristallisation aus Aethylacetat erhält man 0,86 g (90 %) (20S)-20-Methylpregna-5,16-dien-3β,21-diol, Smp. 182-187 °C.

Beispiel 10

Einer Lösung von 1,80 g (6,0 mMol) (Z)-Pregna-5,17(20)-dien-3β-ol in 8 ml Pyridin werden unter einer Stickstoffatmosphäre 1,26 g (6,6 mMol) und dann 1,03 g (5,4 mMol) Paratoluolsulfonylchlorid zugesetzt. Das Gemisch wird gerührt und dann in 150 ml einer auf 7° abgekühlten Lösung von 5 % Natriumbicarbonat unter Rühren geschüttet. Der Niederschlag wire abfiltriert, der Filterkuchen mit Wasser gewaschen und getrocknet. Man erhält 2,50 g (92 %) rohes (Z)-Pregna-5,17(20)-dien 3β-ol-p-toluolsulfonat, das in der nächsten Verfahrensstufe verwendet werden kann. Eine analytische Probe wird aus Hexan umkristallisiert, Smp. 119-119,3 °C.

Beispiel 11

Einer Lösung von 11,3 g (25 mMol) (Z)-Pregna-5,17(20)-dien-3β-ol-p-toluolsulfonat, 7,51 g (0,25 Mol) Paraformaldehyd und 50 ml (0,53 Mol) Essigsäureanhydrid in 100 ml Methylenchlorid werden unter Rühren 25 ml einer Lösung von Bortrifluoridätherat in Methylenchlorid (1 : 90, v/v) zugesetzt. Die trübe Lösung wird filtriert und dem Filtrat werden unter Rühren 1 000 ml gesättigte Natriumbicarbonatlösung zugesetzt. Die Suspension wird mit Methylenchlorid extrahiert, mit Wasser gewaschen, getrocknet und eingedampft. Man erhält 14,5 g rohes (20S)-20-Methylpregna-5,16-dien-3,21-diol-21-acetat-3-p-toluolsulfonat, das in der nächsten Stufe verwendet werden kann. Eine analytische Probe wird aus Hexan umkristallisiert, Smp. 109-110 °C.

Beispiel 12

Einer Lösung von 11,0 g (19 mMol) (20S)-20-Methylpregna-5,16-dien-3,21-diol-21-acetat-3-p-toluolsulfonat, 4,6 ml wasserfreies Pyridin und 70 ml Methanol wird bei Rückflusstemperatur erhitzt und dann eingedampft. Man setzt 50 ml Toluol zu und dampft ein. Der Rückstand wird mit Hexan zerrieben und filtriert. Der Filterkuchen wird mit Hexan gewaschen, das Filtrat eingedampft und eine Lösung des Rückstandes in Hexan mit 4 : 1 Hexan-Aethylacetat als Elutionsmittel auf Silicagel chromatographiert. Man erhält 7,31 g rohes Produkt und, nach Hochdruckflüssigchromatographie mit 10 : 1 Hexan-Aethylacetat, 4,84 g (66 %) amorphes (20S)-3β,5α,6β-Methoxy-20-methyl-3,5-cyclopregn-16-en-21-ol-acetat.

Beispiel 13

Einer Suspension von 380 mg (1,0 mMol) (20S)-3β,5α,6β-Methoxy-20-methyl-3,5-cyclopregn-16-en-21-ol-acetat, 100 mg (1,2 mMol) Natriumbicarbonat, 50 mg 5 % Platin auf Aktivkohle und 25 ml absolutem Aethanol wird unter einer Wasserstoffatmosphäre (Aufnahme 20 ml, Theorie 24 ml) gerührt. Der Katalysator wird abfiltriert und mit Aethanol gewaschen. Das Filtrat wird eingedampft und der Rückstand nach Zusatz von Wasser mit Methylenchlorid extrahiert. Die organische Phase wird mit Wasser gewaschen, getrocknet und eingedampft. Man erhält 352 mg (90 %) (20S)-3β,5α,6β-Methoxy-20-methyl-3,5-cyclopregnan-21-ol-acetat, Smp. 116-123 °C.

Beispiel 14

Eine Lösung von (Z)-1α,3β-Pregnen-5,17(20)-dien-1,3-diol, 10,0 g (31,6 mMol) in 100 ml Pyridin wird unter einer Argonatmosphäre gerührt und in einem Eisbad abgekühlt. Dem Gemisch werden 12,5 ml (88,1 mMol) Trifluoressigsäureanhydrid zugesetzt und das Gemisch wird gerührt und dann mit 52 ml Aethylacetat verdünnt. Die organische Phase wird mit Salzsäure, dann nacheinander mit 300 ml Wasser/gesättigter Natriumchloridlösung (1 : 1) und 10 ml Methanol, mit 100 ml Wasser/gesättigter Natriumbicarbonatlösung (10 : 1) und mit 150 ml Wasser gewaschen. Nach Trocknen wird das Lösungsmittel entfernt und man erhält 15,48 g rohes (Z)-1α,3β-Pregnen-5,17(20)-dien-1,3-diol-1,3-bistrifluoracetat als gelbes Oel.

Beispiel 15

15,48 g (30,4 mMol) (Z)-1α,3β-Pregnen-5,17(20)-dien-1,3-diol-1,3-bistrifluoracetat werden in 155 ml Methylenchlorid gelöst. Man setzt 0,96 g (1,05 Aequivalent) Paraformaldehyd und 31 ml einer Lösung von Bortrifluoridätherat in Methylenchlorid zu. Die Lösung wird bei Raumtemperatur unter einer Argonatmosphäre gerührt. Die organische Phase wird nacheinander mit Wasser/gesättigter Natriumbicarbonatlösung (75 : 5), mit Wasser und mit gesättigter Natriumchloridlösung gewaschen und dann getrocknet. Nach Entfernung des Lösungsmittels erhält man 15,81 g (20S)-20-Methyl-1α,3β-bistrifluoracetoxypregna-5,16-dien-21-ol als bräunlichen Schaum.

Beispiel 16

Eine Lösung von 15,81 g (20S)-20-Methyl-1α,3β-bistrifluoracetoxypregna-5,16-dien-21-ol in 50 ml Aethylacetat wird mit 1,0 g Aktivkohle bei Raumtemperatur behandelt. Nach Filtration wird die Lösung mit 170 ml Aethylacetat verdünnt und bei Raumtemperatur unter atmosphärischem Druck in Gegenwart von 1,63 g 5 % Pt/C hydriert. Nach einer Gesamtaufnahme von 741 ml Wasserstoff (Theorie 706 ml) wird der Katalysator abfiltriert und das Lösungsmittel entfernt. Man erhält 15,42 g (20S)-1α,3β-Bistrifluoracetoxy-20-methylpregn-5-en-21-ol als weisser Schaum.

Beispiel 17

15,42 g (28,5 mMol) (20S)-1α,3β-Bistrifluoracetoxy-20-methylpregn-5-en-21-ol werden in 70 ml Pyridin gelöst, abgekühlt und im Eisbad unter Argonatmosphäre gerührt und dann mit 8,51 g (44,6 mMol) p-Toluolsulfonylchlorid versetzt. Dann werden 150 ml Eis und Wasser zugesetzt und das Produkt wird mit Aethylacetat extrahiert. Der organische Extrakt wird nacheinander mit 2N Salzsäurelösung, mit gesättigter Natriumbicarbonatlösung und mit Wasser und gesättigter Natriumchloridlösung gewaschen. Nach Trocknen und Abdampfen des Lösungsmittels erhält man 18,03 g (20S)-20-Methyl-1α,3β-bistrifluoracetoxypregn-5-en-21-ol-p-toluolsulfonat in Form eines weissen Schaums.

Beispiel 18

18,03 g (25,9 mMol) (20S)-20-Methyl-1α,3β-bistrifluoracetoxypregn-5-en-21-p-toluolsulfonat werden in 150 ml Methanol gelöst und im Wasserbad unter einer Argonatmosphäre unter Rühren abgekühlt. 20 ml einer 20 %-igen Kaliumcarbonatlösung werden zugesetzt. Die Abkühlung wird unterbrochen und die Umsetzung bei Raumtemperatur fortgesetzt. Es werden 100 ml Methanol und dann 10 ml einer 40 %-igen Kaliumcarbonatlösung zugesetzt. Das Produkt wird durch Zusatz von Wasser und Abkühlen kristallisiert. Das Produkt wird filtriert, mit Methanol-Wasser (250 : 130) gewaschen und getrocknet, wobei man 10,05 g (63,3 %) (20S)-20-Methyl-21-(p-toluolsulfonyloxy) pregn-5-en-1α,3β-diol, Smp. 91-99 °C erhält.

**Patentansprüche** (für die Vertragsstaaten : BE, CH, DE, FR, GB, IT, LI, NL)

1. Verfahren zur Herstellung einer Verbindung der Formel

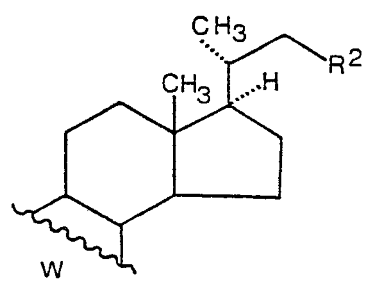

(I)

worin W eine Gruppierung der Formel

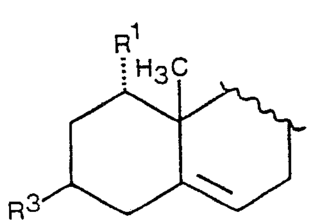

W¹

oder

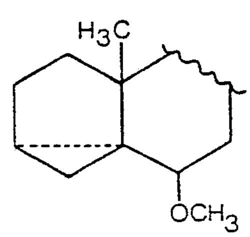

W²

$R^1$ Wasserstoff, Hydroxy oder Acyloxy, $R^2$ und $R^3$ Hydroxy oder Acyloxy, wobei wenn $R^2$ Acyloxy ist $R^1$ und $R^3$ von Hydroxy verschieden sind, welches dadurch gekennzeichnet ist, das man

A) eine Verbindung der Formel

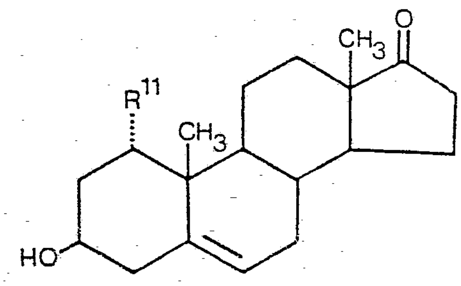

(II)

worin $R^{11}$ Wasserstoff oder Hydroxy ist, in einer Wittig-Reaktion mit einem Aethyltriphenylphosphoniumhalogenid zu einer Verbindung der Formel

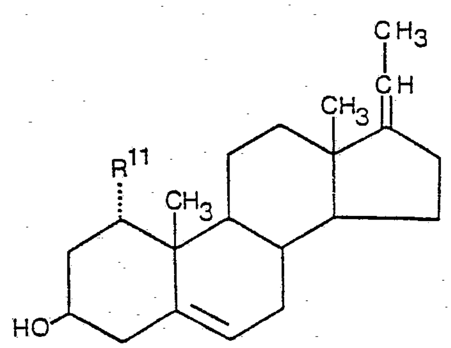

(IIIa)

worin $R^{11}$ die obige Bedeutung hat, umsetzt,

B) a) gewünschtenfalls die in einem Steroid der Formel IIIa enthaltenen 1α- und/oder 3β-Hydroxygruppen acyliert und gewünschtenfalls die Acylgruppe in 3-Stellung in einem erhaltenen diacylierten Steroid der Formel IIIa selektiv hydrolysiert, b) gewünschtenfalls die Hydroxygruppe in einer Verbindung der Formel IIIa, worin $R^{11}$ Wasserstoff ist, zu einer reaktionsfähigeren Abgangsgruppe umwandelt,

C) das Produkt von Stufe A) oder B) der Formel

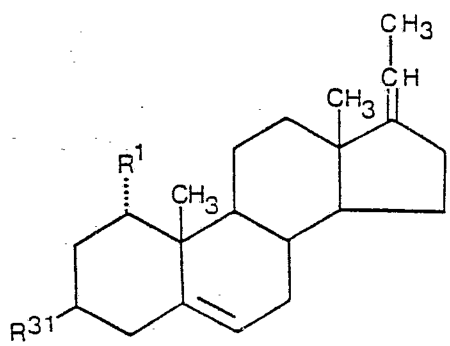

(IIIb)

worin $R^1$ die obige Bedeutung hat und $R^{31}$ Hydroxy, Acyloxy oder, falls $R^1$ Wasserstoff ist, auch eine Abgangsgruppe darstellt, mit Formaldehyd gewünschtenfalls in Gegenwart eines Acylierungsmitels zu einer Verbindung der Formel

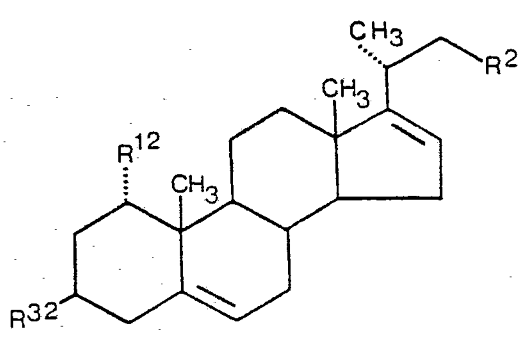

(IVa)

worin entweder $R^2$ Hydroxy und $R^{12}$ und $R^{32}$ die Bedeutungen von $R^1$ bzw. $R^{31}$ oder $R^2$ Acyloxy, $R^{12}$ Wasserstoff oder Acyloxy und $R^{32}$ Acyloxy oder eine Abgangsgruppe darstellen, umsetzt,

D) gewünschtenfalls eine Vergindung der Formel

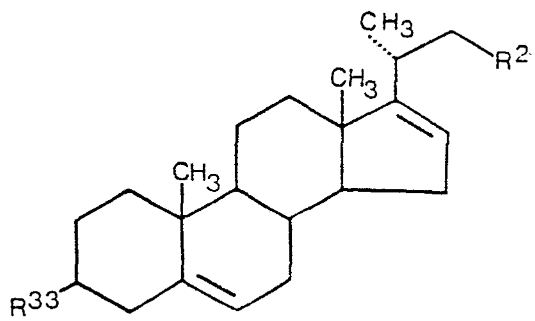

(IVb)

worin $R^2$ die obige Bedeutung hat und $R^{33}$ eine Abgangsgruppe ist, mit einer schwachen Base und Methanol zu einer Verbindung der Formel

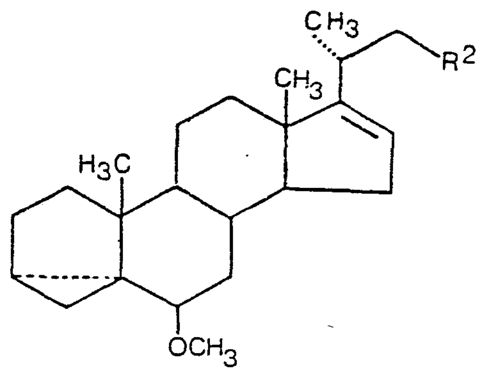

(IVc)

worin $R^2$ die obige Bedeutung hat, umsetzt und

E) das Produkt von Stufe C) oder D) der Formel

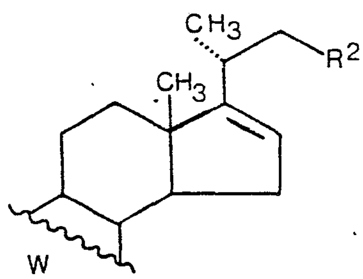

(IV)

worin W und $R^2$ die obige Bedeutung haben, katalytisch hydriert.

2. Verfahren nach Anspruch 1, dadurch gekennzeichnet, dass man die Stufe B) mit einer Verbindung die fähig ist, den Aldehyd in situ zu erzeugen, vorzugsweise Paraformaldehyd, und in Gegenwart von Bortrifluoridätherat durchführt und der Hydrierungskatalysator in Stufe E) 5 % Platin auf Aktivkohle ist.

3. Verfahren nach Anspruch 1, dadurch gekennzeichnet, dass die in Stufe B) in einer Verbindung der Formel IIIa eingesetzte Abgangsgruppe eine Niederalkylsulfonyloxy- oder Arylsulfonyloxygruppe, vorzugsweise p-Toluolsulfonyloxy ist.

4. Eine Verbindung der Formel

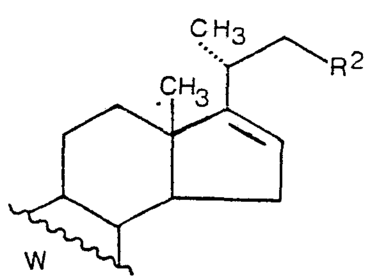

(IV)

worin W eine der Gruppen der Formel

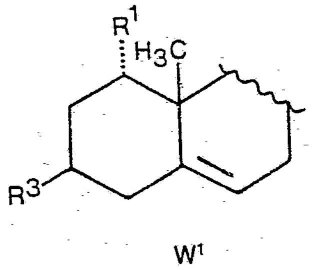

oder

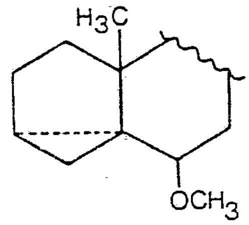

$W^1$ $W^2$

$R^1$ Wasserstoff, Hydroxy oder Acyloxy, $R^2$ und $R^3$ Hydroxy oder Acyloxy, wobei, wenn $R^2$ Acyloxy ist, $R^1$ und $R^3$ von Hydroxy verschieden ist.

5. Die Verbindungen :

(20S)-20-Methylpregna-5,16-dien-3β,21-diol,
(20S)-20-Methylpregna-5,16-dien-3β,21-diol-3-acetat,
(20S)-20-Methylpregna-5,16-dien-1α,3β,21-triol,
(20S)-20-Methylpregna-5,16-dien-1α,3β,21-triol-3-acetat,
(20S)-20-Methylpregna-5,16-dien-1α,3β,21-triol-1-acetat,
(20S)-20-Methylpregna-5,16-dien-1α,3β,21-triol-1,3-diacetat,
(20S)-20-Methyl-1α,3β-bistrifluoracetoxy-pregna-5,16-dien-21-ol,
(20S)-20-Methylpregna-5,16-dien-3β,21-diol-diacetat,
(20S)-20-Methylpregna-5,16-dien-1α,3β,21-triol-1,3,21-triacetat,
(20S)-20-Methylpregna-5,16-dien-3,21-diol-21-acetat-3-p-toluolsulfonat,
(20S)-3β,5α,6β-Methoxy-20-methyl-3,5-cyclopregn-16-en-21-ol und
(20S)-3β,5α,6β-Methoxy-20-methyl-3,5-cyclopregn-16-en-21-ol-acetat.

6. Eine Verbindung der Formel

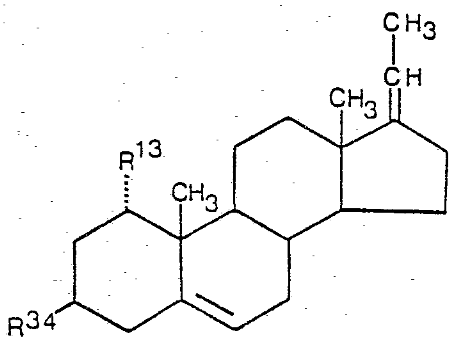

(IIIc)

worin $R^{13}$ und $R^{34}$ Hydroxy oder Trifluoracetoxy, oder $R^{13}$ Wasserstoff und $R^{34}$ Halogen, Niederalkylsulfonyloxy oder Arylsulfonyloxy ist.

7. Die Verbindung (Z)-1α,3β-Pregna-5,17(20)-dien-1,3-diol, (Z)-Pregna-5,17(20)-dien-3β-ol-p-toluolsulfonat und (Z)-1α,3β-Pregna-5,17(20)-dien-1,3-diol-1,3-bistrifluoracetat.

8. Die Verbindungen (20S)-1α,3β-Bistrifluoracetoxy-20-methylpregn-5-en-21-ol und (20S)-20-Methyl-1α,3β-bistrifluoracetoxypregn-5-en-21-ol-p-toluolsulfonat.

**Patentansprüche** (für den Vertragsstaat AT)

1. Verfahren zur Herstellung einer Verbindung der Formel

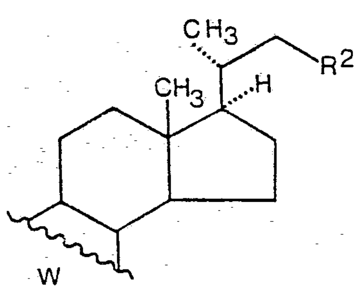

(I)

worin W eine Gruppierung der Formel

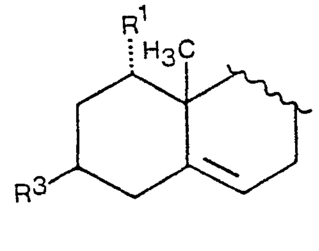

oder

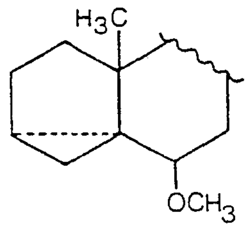

W¹ W²

$R^1$ Wasserstoff, Hydroxy oder Acyloxy, $R^2$ und $R^3$ Hydroxy oder Acyloxy, wobei wenn $R^2$ Acyloxy ist $R^1$ und $R^3$ von Hydroxy verschieden sind, welches dadurch gekennzeichnet ist, dass man

A) eine Verbindung der Formel

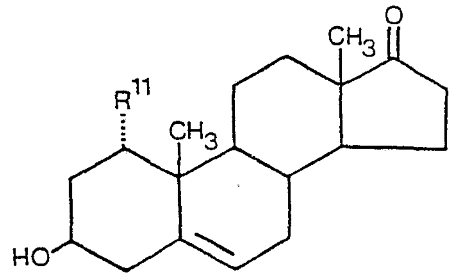

(II)

worin $R^{11}$ Wasserstoff oder Hydroxy ist, in einer Wittig-Reaktion mit einem Aethyltriphenylphosphoniumhalogenid zu einer Verbindung der Formel

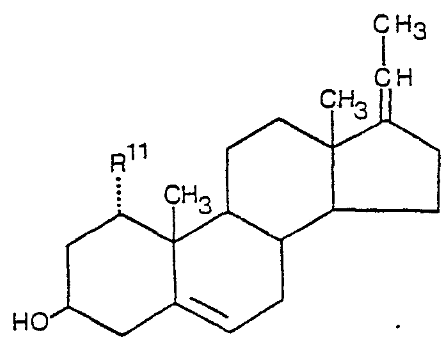

(IIIa)

worin $R^{11}$ die obige Bedeutung hat, umsetzt,

B) a) gewünschtenfalls die in einem Steroid der Formel IIIa enthaltenen 1α- und/oder 3β-Hydroxygruppen acyliert und gewünschtenfalls die Acylgruppe in 3-Stellung in einem erhaltenen diacylierten Steroid der Formel IIIa selektiv hydrolysiert, b) gewünschtenfalls die Hydroxygruppe in einer Verbindung der Formel IIIa, worin $R^{11}$ Wasserstoff ist, zu einer reaktionsfähigeren Abgangsgruppe umwandelt,

C) das Produkt von Stufe A) oder B) der Formel

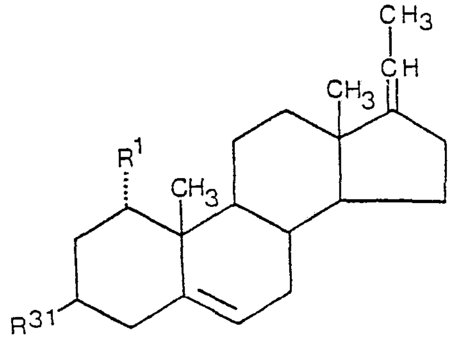

(IIIb)

worin $R^1$ die obige Bedeutung hat und $R^{31}$ Hydroxy, Acyloxy oder, falls $R^1$ Wasserstoff ist, auch eine Abgangsgruppe darstellt, mit Formaldehyd gewünschtenfalls in Gegenwart eines Acylierungsmittels zu einer Verbindung der Formel

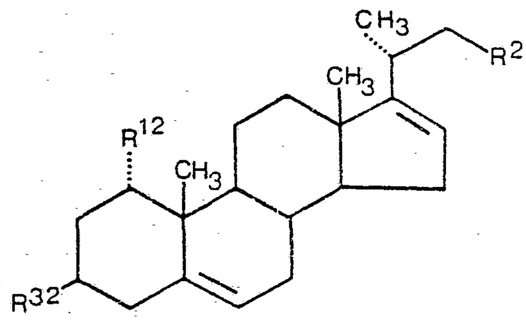

(IVa)

worin entweder $R^2$ Hydroxy und $R^{12}$ und $R^{32}$ die Bedeutungen von $R^1$ bzw. $R^{31}$ oder $R^2$ Acyloxy, $R^{12}$ Wasserstoff oder Acyloxy und $R^{32}$ Acyloxy oder eine Abgangsgruppe darstellen, umsetzt,

D) gewünschtenfalls eine Verbindung der Formel

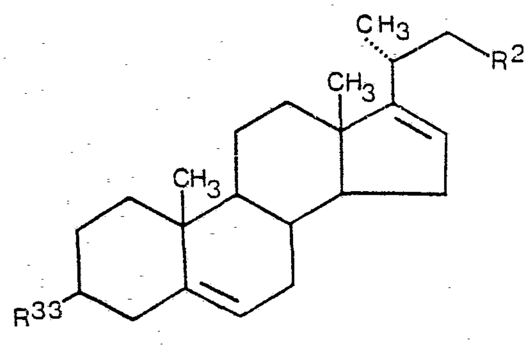

(IVb)

worin $R^2$ die obige Bedeutung hat und $R^{33}$ eine Abgangsgruppe ist, mit einer schwachen Base und Methanol zu einer Verbindung der Formel

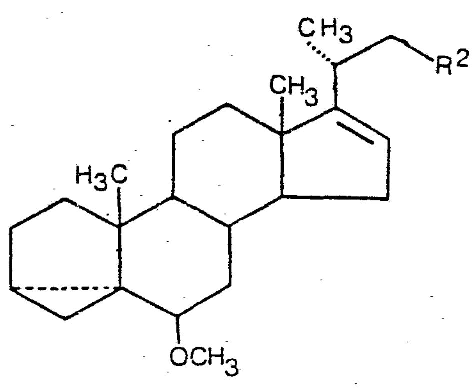

(IVc)

worin $R^2$ die obige Bedeutung hat, umsetzt und

E) das Produkt von Stufe C) oder D) der Formel

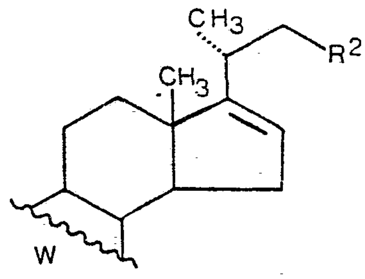

(IV)

worin W und $R^2$ die obige Bedeutung haben, katalytisch hydriert.

2. Verfahren nach Anspruch 1, dadurch gekennzeichnet, dass man die Stufe B) mit einer Verbindung die fähig ist, den Aldehyd in situ zu erzeugen, vorzugsweise Paraformaldehyd, und in Gegenwart von Bortrifluoridätherat durchführt und der Hydrierungskatalysator in Stufe E) 5 % Platin auf Aktivkohle ist.

3. Verfahren nach Anspruch 1, dadurch gekennzeichnet, dass die in Stufe B) in einer Verbindung der Formel IIIa eingesetzte Abgangsgruppe eine Niederalkylsulfonyloxy- oder Arylsulfonyloxygruppe, vorzugsweise p-Toluolsulfonyloxy ist.

**Claims** (for the Contracting States : BE, CH, DE, FR, GB, IT, LI, NL)

1. A process for the preparation of a compound of formula

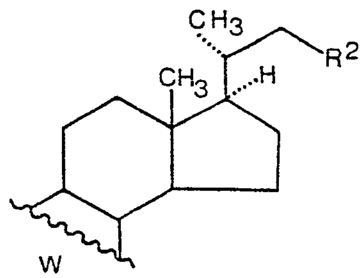

(I)

wherein W is one of the moieties

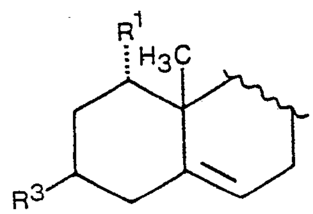

$W^1$

or

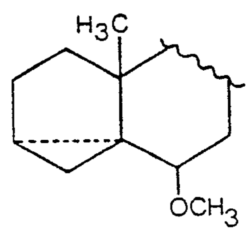

$W^2$

$R^1$ is hydrogen, hydroxy or acyloxy, $R^2$ and $R^3$ are hydroxy or acyloxy, $R^1$ and $R^3$ being different from hydroxy when $R^2$ is acyloxy, which process comprises

A) reacting a compound of formula

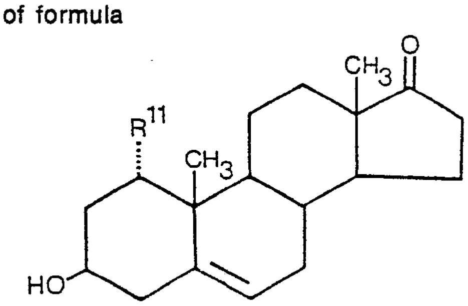

(II)

wherein $R^{11}$ is hydrogen or hydroxy, in a Wittig reaction with an ethyltriphenylphosphonium halide, to yield a compound of formula

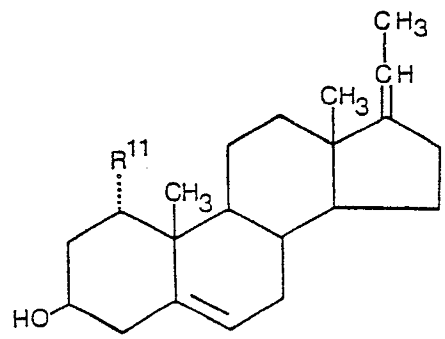

(IIIa)

wherein $R^{11}$ is as above,

B) a) if desired, acylating the 1α- and/or 3β-hydroxy groups contained in a steroid of formula IIIa and if desired selectively hydrolysing the acyl group in the 3-position of an obtained diacylated steroid of formula IIIa, b) if desired, converting the hydroxy group contained in a compound of formula IIIa wherein $R^{11}$ is hydrogen, to a more reactive leaving group,

C) reacting a product of step A) or B) of formula

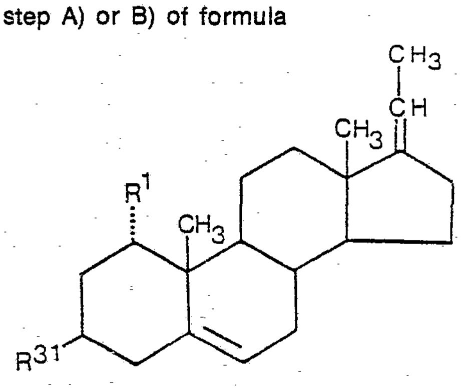

(IIIb)

wherein $R^1$ is as above and $R^{31}$ is hydroxy, acyloxy or, where $R^1$ is hydrogen, is also a leaving group, with formaldehyde, if desired in the presence of an acylating agent, to yield a compound of formula

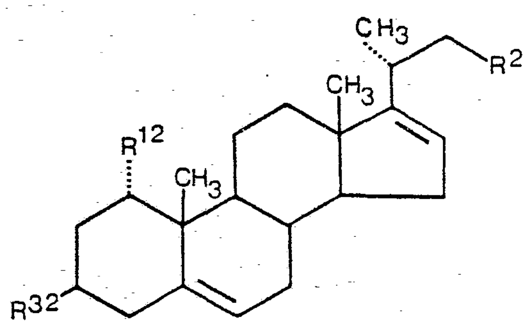

(IVa)

wherein either $R^2$ is hydroxy and $R^{12}$ and $R^{32}$ have the significance given for $R^1$ and $R^{31}$, respectively, or $R^2$ is acyloxy, $R^{12}$ is hydrogen or acyloxy and $R^{32}$ is acyloxy or a leaving group,

D) if desired, reacting a compound of formula

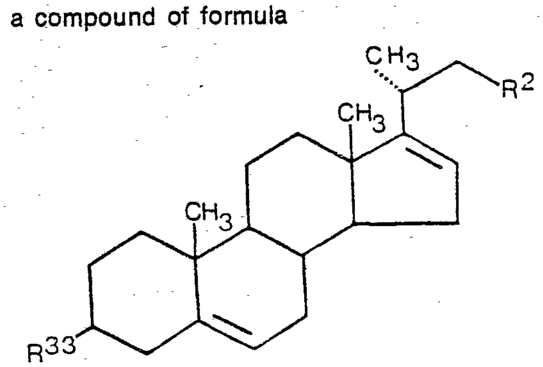

(IVb)

wherein $R^2$ is as above and $R^{33}$ is a leaving group, with a weak base and methanol, to yield a compound of formula

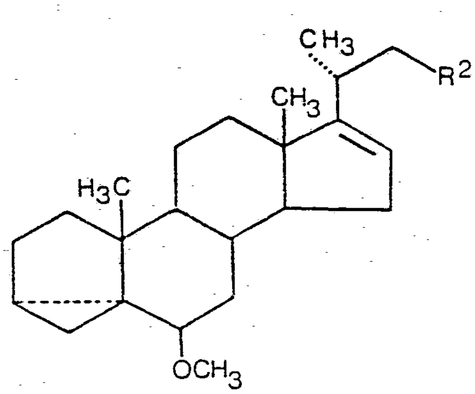

(IVc)

wherein $R^2$ is as above, and

E) catalytically hydrogenating a product of step C) or D) of formula

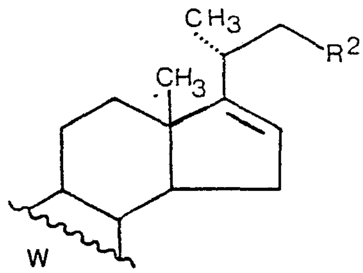

(IV)

wherein W and $R^2$ are as above.

2. A process as in claim 1, wherein step B) is carried out with a compound capable of generating formaldehyde in situ, preferably paraformaldehyde, and in the presence of a Lewis or protic acid, preferably boron trifluoride etherate, and wherein the hydrogenation catalyst in step E) is 5 % platinum on charcoal.

3. A process as in claim 1, wherein the leaving group introduced in a compound of formula IIIa in step B), is a lower alkylsulfonyloxy or arylsulfonyloxy group, preferably p-toluenesulfonyloxy.

4. A compound of the formula

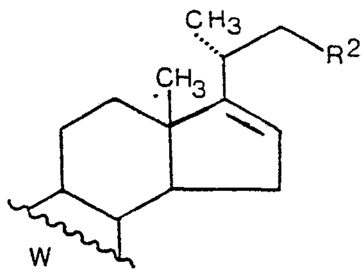

(IV)

wherein W is one of the moieties

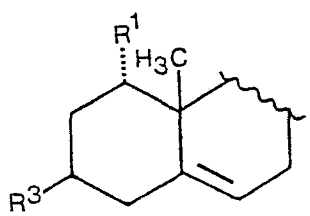

or

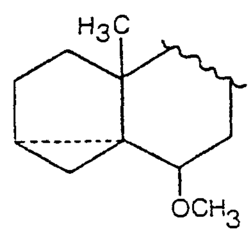

$W^1$ $W^2$

$R^1$ is hydrogen, hydroxy or acyloxy, $R^2$ and $R^3$ are hydroxy or acyloxy, $R^1$ and $R^3$ being different from hydroxy when $R^2$ is acyloxy.

5. The compounds

(20S)-20-methylpregna-5,16-diene-3β,21-diol,
(20S)-20-methylpregna-5,16-diene-3β,21-diol 3-acetate,
(20S)-20-methylpregna-5,16-diene-1α,3β,21-triol,
(20S)-20-methylpregna-5,16-diene-1α,3β,21-triol 3-acetate,
(20S)-20-methylpregna-5,16-diene-1α,3β,21-triol 1-acetate,
(20S)-20-methylpregna-5,16-diene-1α,3β,21-triol 1,3-diacetate,
(20S)-20-methyl-1α,3β-bistrifluoroacetoxy-pregna-5,16-diene-21-ol,
(20S)-20-methylpregna-5,16-diene-3β,21-diol diacetate,
(20S)-20-methylpregna-5,16-diene-1α,3β,21-triol 1,3,21-triacetate,
(20S)-20-methylpregna-5,16-dien-3,21-diol 21-acetate 3-p-toluenesulfonate,
(20S)-3β,5α,6β-methoxy-20-methyl-3,5-cyclopregn-16-en-21-ol and
(20S)-3β,5α,6β-methoxy-20-methyl-3,5-cyclopregn-16-en-21-ol acetate.

6. A compound of the formula

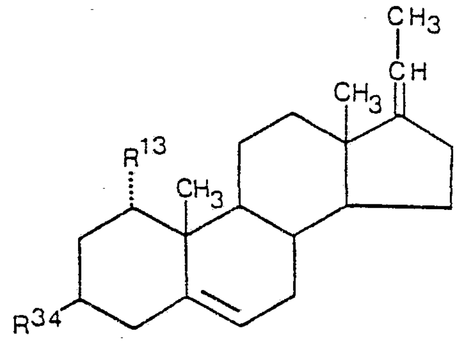

(IIIc)

wherein $R^{13}$ and $R^{34}$ are hydroxy or trifluoroacetoxy, or $R^{13}$ is hydrogen and $R^{34}$ is halogen, lower alkylsulfonyloxy or arylsulfonyloxy.

7. The compounds (Z)-1α,3β-pregna-5,17(20)-diene-1,3-diol, (Z)-pregna-5,17(20)-diene-3β-ol p-toluenesulfonate and (Z)-1α,3β-pregna-5,17(20)-diene-1,3-diol 1,3-bistrifluoroacetate.

8. The compounds (20S)-1α,3β-bistrifluoroacetoxy-20-methylpregn-5-en-21-ol and (20S)-20-methyl-1α,3β-bistrifluoroacetoxypregn-5-en-21-ol p-toluenesulfonate.

**Claims** (for the Contracting State AT)

1. A process for the preparation of a compound of formula

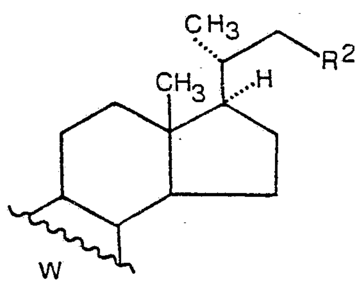

(I)

wherein W is one of the moieties

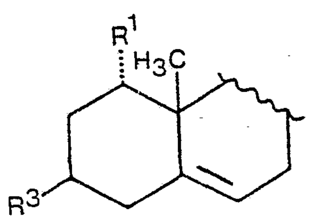

or

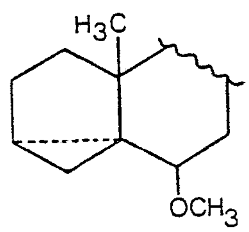

W¹ W²

$R^1$ is hydrogen, hydroxy or acyloxy, $R^2$ and $R^3$ are hydroxy or acyloxy, $R^1$ and $R^3$ being different from hydroxy when $R^2$ is acyloxy, which process comprises

A) reacting a compound of formula

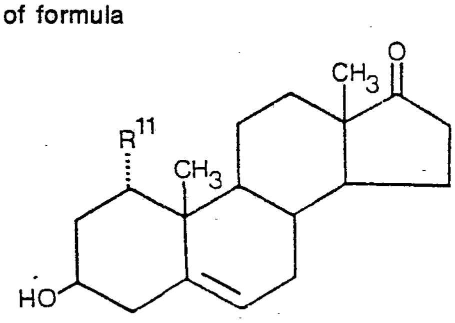

(II)

wherein $R^{11}$ is hydrogen or hydroxy, in a Wittig reaction with an ethyltriphenylphosphonium halide, to yield a compound of formula

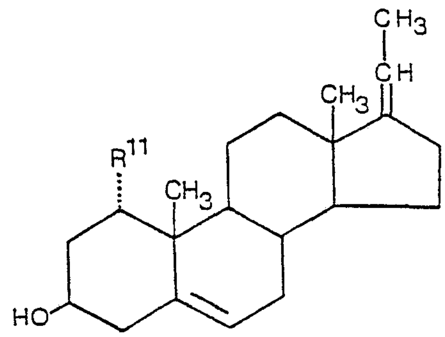

(IIIa)

wherein $R^{11}$ is as above,

B) a) if desired, acylating the 1α- and/or 3β-hydroxy groups contained in a steroid of formula IIIa and if desired selectively hydrolysing the acyl group in the 3-position of an obtained diacylated steroid of formula IIIa, b) if desired, converting the hydroxy group contained in a compound of formula IIIa wherein $R^{11}$ is hydrogen, to a more reactive leaving group,

C) reacting a product of step A) or B) of formula

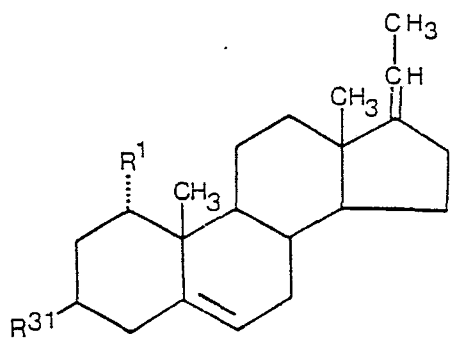

(IIIb)

wherein $R^1$ is as above and $R^{31}$ is hydroxy, acyloxy or, where $R^1$ is hydrogen, is also a leaving group, with formaldehyde, if desired in the presence of an acylating agent, to yield a compound of formula

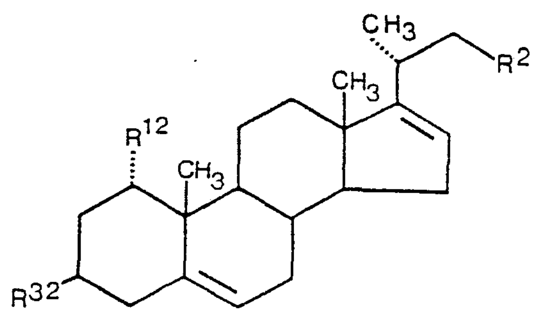

(IVa)

wherein either $R^2$ is hydroxy and $R^{12}$ and $R^{32}$ have the significance given for $R^1$ and $R^{31}$, respectively, or $R^2$ is acyloxy, $R^{12}$ is hydrogen or acyloxy and $R^{32}$ is acyloxy or a leaving group,

D) if desired, reacting a compound of formula

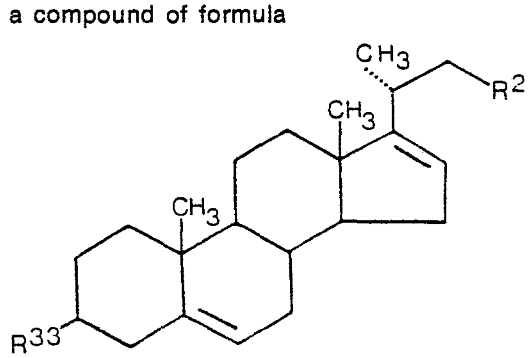

(IVb)

wherein $R^2$ is as above and $R^{33}$ is a leaving group, with a weak base and methanol, to yield a compound of formula

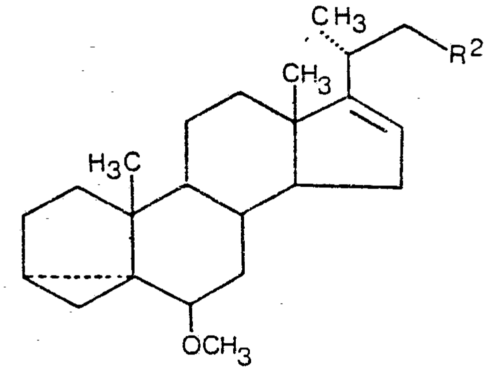

(IVc)

wherein $R^2$ is as above, and

E) catalytically hydrogenating a product of step C) or D) of formula

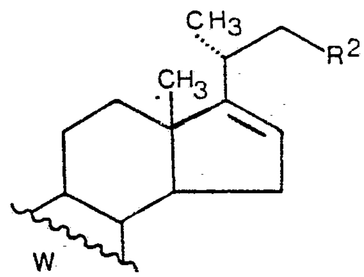

wherein W and $R^2$ are as above.

2. A process as in claim 1, wherein step B) is carried out with a compound capable of generating formaldehyde in situ, preferably paraformaldehyde, and in the presence of a Lewis or protic acid, preferably boron trifluoride etherate, and wherein the hydrogenation catalyst in step E) is 5 % platinum on charcoal.

3. A process as in claim 1, wherein the leaving group introduced in a compound of formula IIIa in step B), is a lower alkylsulfonyloxy or arylsulfonyloxy group, preferably p-toluenesulfonyloxy.

**Revendications** (pour les Etats contractants : BE, CH, DE, FR, GB, IT, LI, NL)

1. Procédé de préparation d'un composé de formule

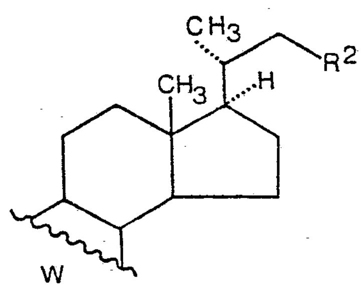

(I)

dans laquelle W représente un groupement de formule

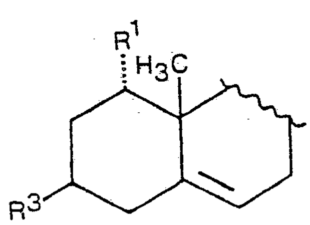

ou

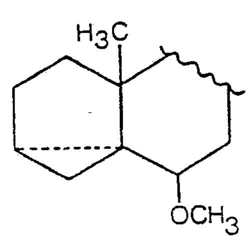

$W^1$ $W^2$

$R^1$ représente l'hydrogène, un groupe hydroxy ou acyloxy, $R^2$ et $R^3$ représentent des groupes hydroxy ou acyloxy, étant spécifié que lorsque $R^2$ représente un groupe acyloxy, $R^1$ et $R^3$ ne peuvent représenter des groupes hydroxy, caractérisé en ce que

A) on fait réagir un composé de formule

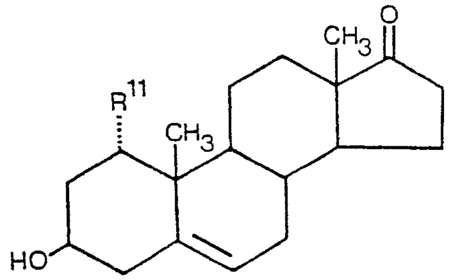

(II)

dans laquelle $R^{11}$ représente l'hydrogène ou un groupe hydroxy, dans une réaction de Wittig avec un halogénure d'éthyltriphénylphosphonium, donnant un composé de formule

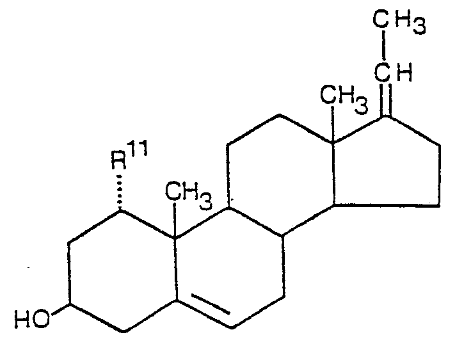

(IIIa)

dans laquelle $R^{11}$ a la signification indiquée ci-dessus,

B) a) si on le désire, on acyle les groupes 1α- et/ou 3β-hydroxy contenus dans un stéroïde de formule IIIa, et si on le désire, on hydrolyse sélectivement le groupe acyle en position 3 d'un stéroïde diacylé de formule IIIa ainsi obtenu, b) si on le désire, on convertit le groupe hydroxy d'un composé de formule IIIa dans laquelle $R^{11}$ représente l'hydrogène, en un groupe éliminable plus réactif,

C) on fait réagir le produit du stade A) ou B) de formule

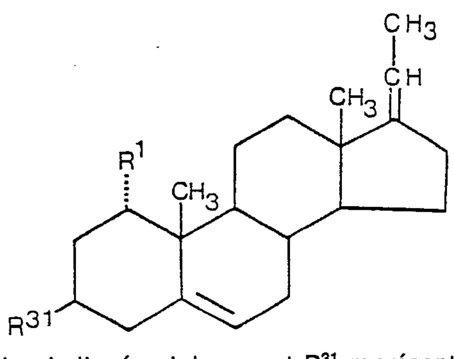

(IIIb)

dans laquelle $R^1$ a la signification indiquée ci-dessus et $R^{31}$ représente un groupe hydroxy, acyloxy ou encore, lorsque $R^1$ représente l'hydrogène, un groupe éliminable, avec le formaldéhyde, si on le désire en présence d'un agent acylant, avec formation d'un composé de formule

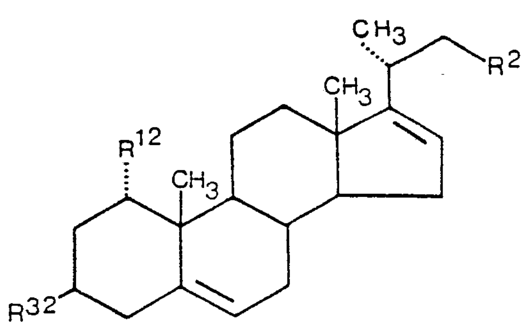

(IVa)

dans laquelle ou bien $R^2$ représente un groupe hydroxy et $R^{12}$ et $R^{32}$ ont les significations respectives de $R^1$ et $R^{31}$ ou bien $R^2$ représente un groupe acyloxy, $R^{12}$ l'hydrogène ou un groupe acyloxy et $R^{32}$ un groupe acyloxy ou un groupe éliminable,

D) si on le désire, on fait réagir un composé de formule

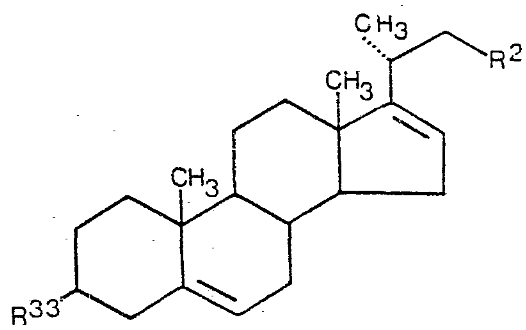

(IVb)

dans laquelle $R^2$ a la signification indiquée ci-dessus et $R^3$ représente un groupe éliminable, avec une base faible et le méthanol, la réaction donnant un composé de formule

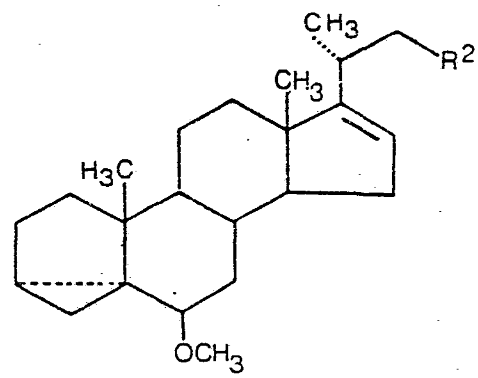

dans laquelle $R^2$ a la signification indiquée ci-dessus, et

E) on soumet le produit du stade C) ou D) de formule

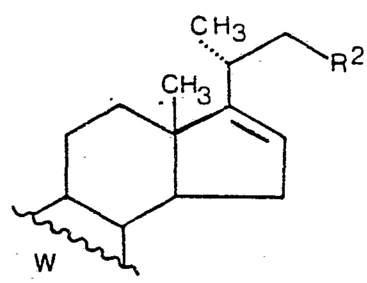

(IV)

dans laquelle W et $R^2$ ont les significations indiquées ci-dessus, à hydrogénation catalytique.

2. Procédé selon la revendication 1, caractérisé en ce que le stade opératoire B) est effectué avec un composé capable de former l'aldéhyde in situ, de préférence le paraformaldéhyde, et en présence d'un éthérate de fluorure de bore, et en ce que le catalyseur d'hydrogénation du stade opératoire E) consiste en platine à 5 % sur charbon actif.

3. Procédé selon la revendication 1, caractérisé en ce que le groupe éliminable utilisé au stade opératoire B) dans un composé de formule IIIa est un groupe alkylsulfonyloxy inférieur ou arylsulfonyloxy, de préférence p-toluène-sulfonyloxy.

4. Un composé de formule

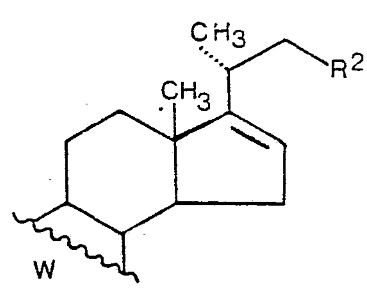

(IV)

dans laquelle W représente l'un des groupes de formule

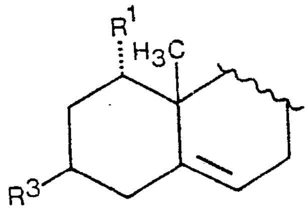

ou

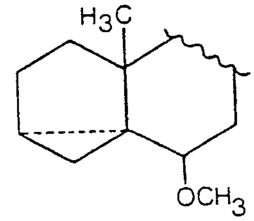

W¹ W²

$R^1$ représente l'hydrogène, un groupe hydroxy ou acyloxy, $R^2$ et $R^3$ représentent des groupes hydroxy ou acyloxy, étant spécifié que lorsque $R^2$ représente un groupe acyloxy, $R^1$ et $R^3$ ne peuvent représenter des groupes hydroxy.

5. Les composés :

(20S)-20-méthylprégna-5,16-diène-3β,21-diol,
3-acétate du (20S)-20-méthylprégna-5,16-diène-3β,21-diol,
(20S)-20-méthylprégna-5,16-diène-1α,3β,21-triol,
3-acétate du (20S)-20-méthylprégna-5,16-diène-1α,3β,21-triol,
1-acétate du (20S)-20-méthylprégna-5,16-diène-1α,3β,21-triol,
1,3-diacétate du (20S)-20-méthylprégna-5,16-diène-1α,3β,21-triol,
(20S)-20-méthyl-1α,3β-bis-trifluoracétoxy-prégna-5,16-diène-21-ol,
diacétate du (20S)-20-méthylprégna-5,16-diène-3β,21-diol,
1,3,21-triacétate du (20S)-20-méthylprégna-5,16-diène-1α,3β,21-triol,
3-p-toluène-sulfonate du 21-acétate du (20S)-20-méthylprégna-5,16-diène-3,21-diol,
(20S)-3β,5α,6β-méthoxy-20-méthyl-3,5-cycloprégna-16-ène-21-ol, et
acétate du (20S)-3β,5α,6β-méthoxy-20-méthyl-3,5-cycloprégna-16-ène-21-ol.

6. Un composé de formule

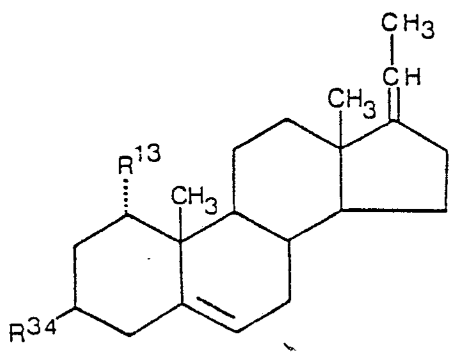

(IIIc)

dans laquelle $R^{13}$ et $R^{34}$ représentent des groupes hydroxy ou trifluoracétoxy, ou bien $R^{13}$ représente l'hydrogène et $R^{34}$ un halogène, un groupe alkylsulfonyloxy inférieur ou arylsulfonyloxy.

7. Les composés (Z)-1α,3β-prégna-5,17(20)-diène-1,3-diol, p-toluènesulfonate du (Z)-prégna-5,17(20)-diène-3β-ol, et 1,3-bis-trifluoracétate du (Z)-1α,3β-prégna-5,17(20)-diène-1,3-diol.

8. Les composés (20S)-1α,3β-bis-trifluoracétoxy-20-méthylprégna-5-ène-21-ol et p-toluène-sulfonate du (20S)-20-méthyl-1α,3β-bis-trifluoracétoxy-prégna-5-ène-21-ol.

**Revendications** (pour l'Etat contractant AT)

1. Procédé de préparation d'un composé de formule

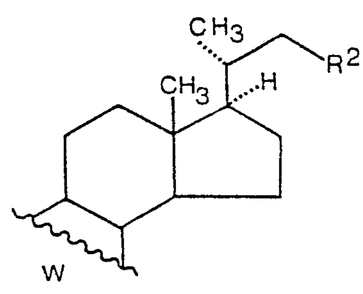

(I)

dans laquelle W représente un groupement de formule

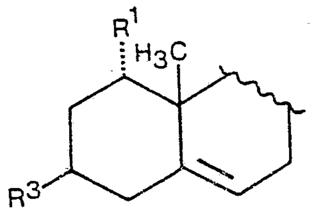

W¹

ou

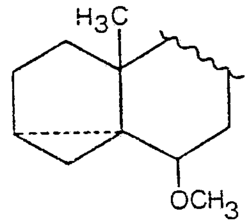

W²

$R^1$ représente l'hydrogène, un groupe hydroxy ou acyloxy, $R^2$ et $R^3$ représentent des groupes hydroxy ou acyloxy, étant spécifié que lorsque $R^2$ représente un groupe acyloxy, $R^1$ et $R^3$ ne peuvent représenter des groupes hydroxy, caractérisé en ce que

A) on fait réagir un composé de formule

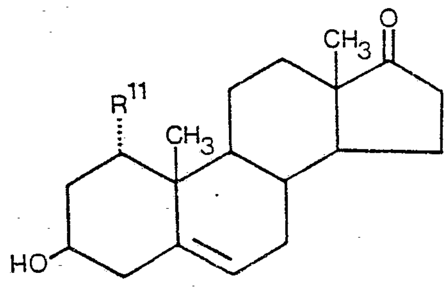

(II)

dans laquelle $R^{11}$ représente l'hydrogène ou un groupe hydroxy, dans une réaction de Wittig avec un halogénure d'éthyltriphénylphosphonium, donnant un composé de formule

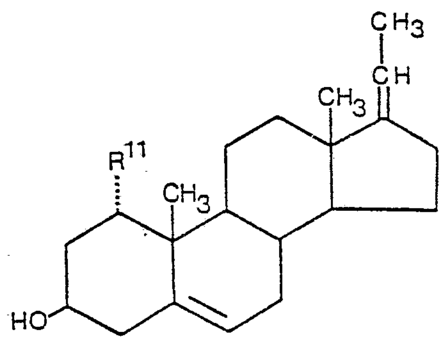

(IIIa)

dans laquelle $R^{11}$ a la signification indiquée ci-dessus,

B) a) si on le désire, on acyle les groupes 1α- et/ou 3β-hydroxy contenus dans un stéroïde de formule IIIa, et si on le désire, on hydrolyse sélectivement le groupe acyle en position 3 d'un stéroïde diacylé de formule IIIa ainsi obtenu, b) si on le désire, on convertit le groupe hydroxy d'un composé de formule IIIa dans laquelle $R^{11}$ représente l'hydrogène, en un groupe éliminable plus réactif,

C) on fait réagir le produit du stade A) ou B) de formule

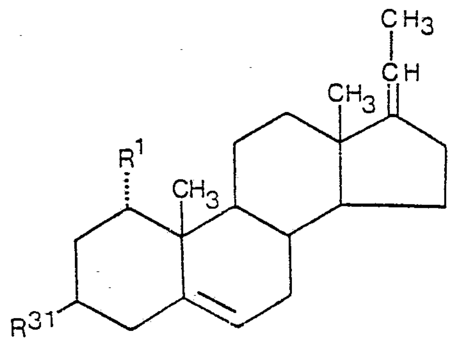

(IIIb)

dans laquelle $R^1$ a la signification indiquée ci-dessus et $R^{31}$ représente un groupe hydroxy, acyloxy ou encore, lorsque $R^1$ représente l'hydrogène, un groupe éliminable, avec le formaldéhyde, si on le désire en présence d'un agent acylant, avec formation d'un composé de formule

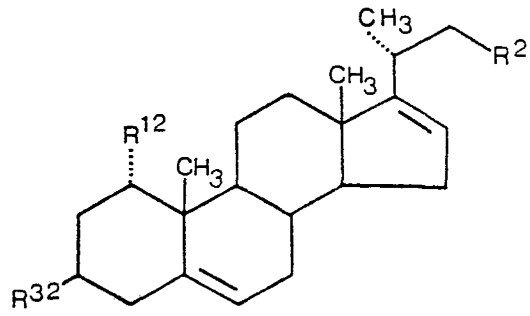

(IVa)

dans laquelle ou bien $R^2$ représente un groupe hydroxy et $R^{12}$ et $R^{32}$ ont les significations respectives de $R^1$ et $R^{31}$ ou bien $R^2$ représente un groupe acyloxy, $R^{12}$ l'hydrogène ou un groupe acyloxy et $R^{32}$ un groupe acyloxy ou un groupe éliminable,

D) si on le désire, on fait réagir un composé de formule

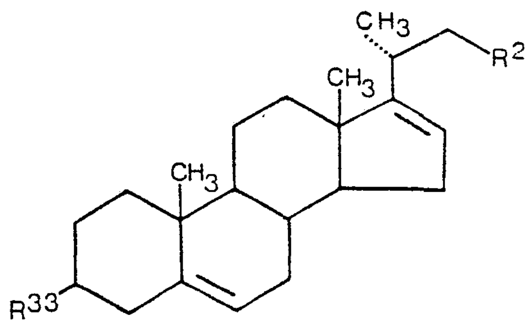

(IVb)

dans laquelle $R^2$ a la signification indiquée ci-dessus et $R^3$ représente un groupe éliminable, avec une base faible et le méthanol, la réaction donnant un composé de formule

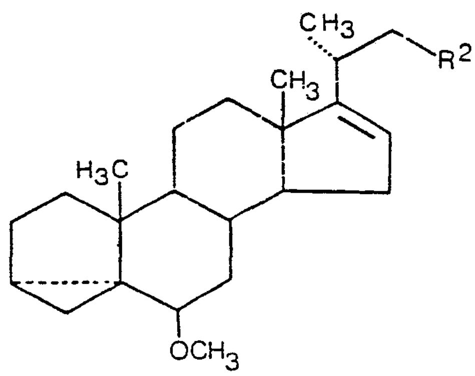

(IVc)

dans laquelle $R^2$ a la signification indiquée ci-dessus, et

E) on soumet le produit du stade C) ou D) de formule

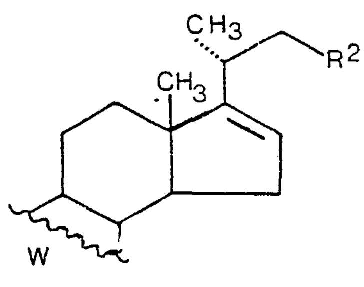

(IV)

dans laquelle W et $R^2$ ont les significations indiquées ci-dessus, à hydrogénation catalytique.

2. Procédé selon la revendication 1, caractérisé en ce que le stade opératoire B) est effectué avec un composé capable de former l'aldéhyde in situ, de préférence le paraformaldéhyde, et en présence d'un éthérate de fluorure de bore, et en ce que le catalyseur d'hydrogénation du stade opératoire E) consiste en platine à 5 % sur charbon actif.

3. Procédé selon la revendication 1, caractérisé en ce que le groupe éliminable utilisé au stade opératoire B) dans un composé de formule IIIa est un groupe alkylsulfonyloxy inférieur ou arylsulfonyloxy, de préférence p-toluène-sulfonyloxy.